# EUROPEAN PATENT SPECIFICATION

(11) **EP 1 143 968 B1**
(45) Date of publication and mention of the grant of the patent: **20.08.2003**
(21) Application number: 00902404.3
(22) Date of filing: 12.01.2000
(51) Int. Cl.: A61K 31/337, A61P 9/10, A61P 35/00

(54) **COMPOSITION AND METHODS FOR ADMINISTRATION OF WATER-INSOLUBLE PACLITAXEL DERIVATIVES**
ARZNEIMITTEL UND VERFAHREN ZUR VERABREICHUNG VON WASSERUNLÖSLICHEN PACLITAXELDERIVATEN
COMPOSITION ET PROCEDES PERMETTANT L'ADMINISTRATION DE DERIVES DE PACLITAXEL NON SOLUBLES DANS L'EAU

(30) Priority: 12.01.1999 US 115609 P; 17.06.1999 US 335442
(43) Date of publication of application: 17.10.2001
(73) Proprietor: Quanam Medical Corporation, Santa Clara, CA 95050 (US)
(72) Inventor: ALVARADO, Angelica, Santa Clara, CA 95051 (US); EURY, Robert, Cupertino, CA 95014 (US); POMERANTSEVA, Irina D., Wakefield, MA 01880 (US); FROIX, Michael, Mountain View, CA 94040 (US)
(74) Representative: VOSSIUS & PARTNER
(86) International application number: US0000832
(87) International publication number: WO00041687

(56) References cited:
- WO-A-95/03795
- WO-A-97/10234
- WO-A-97/45105

## Description

The present invention relates to a composition for administration of water-insoluble derivatives of paclitaxel and to said composition for treating restenosis.

Paclitaxel is an anti-microtubule agent extracted from the needles and bark of the Pacific yew tree, *Taxus brevifolia*, and has been widely studied for use as an anti-neoplastic agent for treatment of cancers, particularly ovarian and breast cancers. More recently, paclitaxel has been recognized for its anti-angiogenesis activity, and its use in treating conditions characterized by vascular growth, such. as tumors, artherosclerosis and restenosis (Kinsella, U.S. Patent No. 5,616,608; Kunz, U.S. Patent No. 5,733,925; Hunter, U.S. Patent No. 5,716,981).

One of the difficulties in the development of the drug for clinical use is its extremely low solubility in water. Further, the compound lacks functional groups that allow for salt formation. As a result, numerous derivatives and analogues of paclitaxel having an improved water soluble have been described (Haugwitz, U.S. Patent No. 5,157,049; WO 97/33552; Mathew, *J. Med. Chem*. 35(1):145 (1992)). Paclitaxel derivatized at several positions, including the C-2, C-7, C-10, C-2' and C-3', has been described (Grover, *et al., Biochemistry* 34(12):3927, (1995); Mathew, *J. Med. Chem*. 35(1):145, (1992); Sengupta, *et al., Biochemistry* 36(17):5179. (1997)). While derivatives having improved water solubility can be readily prepared, the biological activity and stability of the derivatives is less certain.

Another approach to overcoming the poor water solubility of paclitaxel has been to formulate the drug into liposomes (Balasubramanian, *et al*., *Biochemistry* 33(30):8941, (1994); Sharma, *et al., Melanoma Res.* 8(3):240, (1998); Sharma, *et al., Pharm. Res.* 11(6):889, (1994)).

Another approach has been to formulate the drug into a biodegradable implant for local delivery, such as at the site of a tumor resection (Park, *et al., J. Controlled Release* 52(1-2):179, (1998)). For example, U.S. Patent No. 5,733,925 to Kunz describes a dosage form having an agent for inhibiting vascular smooth muscle proliferation, where the dosage form can be a polymer matrix with the therapeutic agent incorporated within. When the agent is soluble in the polymer matrix, the agent is released by diffusion through the matrix and into the surrounding environment. When the agent is nor soluble in the polymer matrix, such as paclitaxel, the dosage form is prepared from a biodegradable polymer matrix, for release of the agent as the matrix degrades.

U.S. Patent No. 5,716,981 to Hunter describes another approach to formulation of the poorly water-soluble paclitaxel into a polymer carrier for *in vivo* use. The compound is combined with a carbohydrate, protein or peptide matrix, and the matrix is combined with the polymer carrier. In this way, the poorly soluble drug is coated or surrounded by a hydrophilic shell, for formulation into the polymer carrier.

WO97/45105 discloses paclitaxel derivatives which may be contained in a polymeric material for the treatment of restenosis. However, the document does not refer to paclitaxel derivatives with a water solubility less than that of pactilaxel.

In summary, the convention in the art is to design a water-soluble derivative or analogue of paclitaxel, and of other compounds, for ease of formulation and administration. The art has not recognized the value in poorly water-soluble paclitaxel derivatives for *in vivo* therapy.

In one aspect, the invention includes a composition for administration of a paclitaxel derivative, comprising a paclitaxel derivative having a water solubility less than that of paclitaxel, and a polymeric material containing the paclitaxel derivative, wherein the polymeric material is in a form selected from the group consisting of a coating; a sleeve; a sheath or an implantable medical device.

In one embodiment, the paclitaxel derivative is a compound derivatized at the 2', 10 or 7 position of taxol. In a preferred embodiment, the paclitaxel derivative is 7-hexanoyl taxol.

In another embodiment, the medical device forms a stent which is suitable for placement in a target lumen, and exemplary polymeric materials are acrylate, methacrylate and polyalkyleneoxide polymers.

In another embodiment, the paclitaxel derivative is incorporated into the polymeric material in particulate form.

In another aspect, the invention includes a composition as described above for treatment of restenosis.

Described herein are polymer compositions for administration of a low solubility by paclitaxel derivative. One exemplary composition is composed of greater than about 40 weight percent of an acrylate monomer and between about 2-40 weight percent of a polyalkyleneoxide monomer; and a paclitaxel derivative having a water solubility less than that of paclitaxel. The monomers, when polymerized, form a polymer in which the paclitaxel derivative can be incorporated, as will be further described below. The polymer composition further includes, in some embodiments, between 3-30 weight percent of a methacrylate.

In another embodiment, the acrylate monomer is butyl acrylate. In still another embodiment, the polyalkyleneoxide monomer is a polyethylene oxide, such as polyethylene oxide monomethyl ether monomethacrylate or polyethyleneglycol monomethacrylate.

The polymer composition, in another embodiment, further includes between 2-15 weight percent of an organic solvent that is miscible with the monomers.

The polymer compositions described are suitable for use in fabricating into a stent for insertion into a target lumen.

In another aspect of the invention, the use of the above composition for the manufacture of a medicament for treating restenosis is described. This includes preparing a pharmaceutical preparation composed of a paclitaxel derivative having a water solubility less than that of paclitaxel, whereby the paclitaxel derivative is incorporated into a polymeric material. The preparation is for administration to a patient in need.

These and other objects and features of the invention will be more fully appreciated when the following detailed description of the invention is read in conjunction with the accompanying drawings.
Fig. 1A shows the structure of paclitaxel with the 2', 7 and 10 carbon positions indicated;
Fig. 1B shows the structure of a paclitaxel derivative formed by replacing the hydroxyl group at the 7-carbon position in taxol with an ester;
Fig. 2 is an illustration of an HPLC trace showing the relative water solubilities of paclitaxel and 7-hexanoyl taxol;
Figs. 3A-3C show a support stent (Fig. 3A) suitable for carrying a polymer sleeve (Fig. 3B) or polymer members (Fig. 3C) containing a water-insoluble paclitaxel derivative or other compound;
Figs. 4A-4C illustrate another embodiment of a support stent in its small, unexpanded condition (Fig. 4A) and in its larger diameter, expanded condition (Fig. 4B) which is suitable for carrying polymer members positioned about the rigid support stent regions (Fig. 4C); and
Figs. 5A-5C illustrate yet another embodiment of a support stent in its small, unexpanded condition (Fig. 5A) and in its larger diameter, expanded condition (Fig. 5B) which is suitable for carrying polymer members about the rigid support stent regions (Fig. 5C).

### I. Definitions

"Acrylate monomer" as used herein refers to a monomer capable of forming a polymer of acrylic acid or its esters with a -(CH₂-CH(COOR))ₙ- structure. The R group is typically a group having between 1-50 carbon atoms, more preferably between 1-20 carbon atoms.

"Acrylate" or "acrylate polymer" refers to a polymer, usually a copolymer, prepared from an acrylate monomer.

"Methacrylate monomer" as used herein refers to a monomer for formation of a polymer of methacrylic acid or its esters with a -(CH₂-C(CH₃)(COOR))ₙ- structure. The R group is typically a group having between 1-50 carbon atoms, more preferably between 1-20 carbon atoms.

"Methacrylate" or "methacrylate polymer'' refers to a polymer, usually a copolymer, prepared from a methacrylate monomer.

"Polyalkyleneoxide" refers to a polymer having the general structure R¹(OCH₂(CHR²OCHR³)CH₂O)ₙR⁴, where the R² and R³ can be H or a C1-C10 alkane, and the end groups R¹ and R⁴ can be H or any suitable end moiety, such as CH3 to give a methoxy, or various ethers. Exemplary polyalkyleneoxides include polyethylene oxide (polyethylene glycol), polyethylene oxide monomethyl ether monomethacrylate, polypropylene glycol.

"Polymer" as used herein refers to homopolymers and copolymers, including random, alternating and block copolymers.

### II. Paclitaxel Derivatives

In one aspect, the invention includes a composition for administration of a paclitaxel derivative, that has poor water solubility, as will be discussed below. Paclitaxel (Taxol®) is an anti-microtubule agent extracted from the needles and bark of the Pacific yew tree, *Taxus brevifolia*. The structure of the drug is shown in Fig. 1A, and the reference numerals 2', 7 and 10 in the figure identify some of the carbon positions on the taxane ring that are known to be suitable for derivatization or modification (see for example U.S. Patent Nos. 5,412,116; 5,629,433; 5,283,253; 5,294,637).

Fig. 1B shows the structure of one example of a paclitaxel derivative suitable for use in the present invention. As seen, the compound in Fig. 1B is modified at the 7-carbon position by replacing the hydroxyl with a hexanoyl ester.

According to an important feature of the invention, the paclitaxel derivative for use in the invention, such as the 7-hexanoyl taxol in Fig. 1B, is less water-soluble than paclitaxel (Fig. 1A). The water solubility of 7-hexanoyl taxol relative to paclitaxel was determined via HPLC analysis using a reverse phase column and illustrations of the HPLC traces are shown in Fig. 2. The column employed was made of silica with the hydrophilic Si-OH functionalities converted to hydrophobic diphenylmethyl functionalities. The mobile phase was acetonitrile/water. In this column, a hydrophilic or water soluble test material is retained primarily in the aqueous mobile phase, resulting in the water soluble compounds eluting from the column earlier than hydrophobic or water insoluble compounds. As seen in Fig. 2, paclitaxel (solid line) has a retention time of 18.5 minutes. The 7-hexanoyl taxol (dashed line) has a retention time of 30.9 minutes. The longer retention time of the 7-hexanoyl taxol, *e.g*., the hydrophobic shift, indicates that the derivative is less water soluble than paclitaxel.

That the 7-hexanoyl taxol is less water soluble than paclitaxel is consistent with the fact that an ester is less water soluble than the individual components. For example, ethyl acetate is an ester of ethanol and acetic acid, both of which are water soluble, but the ethyl acetate ester of the two is not water soluble. Similarly, 7-hexanoyl taxol has an hexanoyl ester at the 7-carbon position. More generally, the invention contemplates 7-position esters of any length, provided the derivative is less water soluble than paclitaxel.

Using such guidance, those of skill in the art can contemplate a variety of derivatives of paclitaxel that are likely to be less water soluble than paclitaxel. The invention contemplates use of such derivatives. Such derivatives can be readily prepared by those of skill in the art.

In studies performed in support of the invention, the *in vitro* cytotoxicity of 7-hexanoyl taxol was compared to paclitaxel. As described in Example 1, LC₅₀ values, which signify the concentration of drug at which 50% of the cells are killed, were determined in five cell lines. The results are shown in Table 1.

**Table 1**

| **Cell Type/Line** | **7-hexanoyl taxol LC**_{**50**} **(µM)** | **Paclitaxel LC**_{**50**} **(µM)** |
|---|---|---|
| colon cancer/HT 29 | 30 | 30 |
| melanoma/M14 | 1.1 | 100 |
| melanoma/SK-MEL-2 | > 100 | 100 |
| non-small cell lung cancer/NCI H226 | > 100 | 70 |
| ovarian cancer/OVCAR-5 | 50 | > 100 |

As seen in Table 1, the 7-hexanoyl taxol was more cytotoxic to melanoma/M14 cells and to ovarian cancer/OVCAR-5 cells. 7-hexanoyl taxol and paclitaxel had equal toxicities to colon cancer/HT 29 cells. Paclitaxel was more cytotoxic to melanoma/SK-MEL-2 cells and to the non-small cell lung cancer cells.

### III. Dosage Forms Containing the Insoluble Derivatives

The water-insoluble paclitaxel derivatives of the invention are contemplated for use in a variety of dosage forms for delivery and treatment of conditions characterized by an undesired proliferation of cells. In particular, atherosclerosis and restenosis are two conditions characterized by abnormal cell proliferation and, in preferred embodiments of the invention, are treated using the water-insoluble derivatives of the invention. Atherosclerosis is a form of chronic vascular injury in which some of the normal vascular smooth muscle cells in the artery wall change and develop "cancer-like" behavior. The cells become abnormally proliferative, invading and spreading into the inner vessel lining, blocking blood flow and making the vessel susceptible to complete blockage by blood clotting.

Restenosis is the recurrence of stenosis or artery stricture after corrective surgery and has been referred to as a form of accelerated atherosclerosis. Restenosis is due to a complex series of fibroproliferative responses to vascular injury and is characterized by vascular smooth muscle proliferation, migration and neo-intimal accumulation.

In a preferred embodiment of the invention, a sustained release dosage form is used to treat restenosis. Restenosis occurs after coronary artery bypass surgery, artherectomy, laser ablation, heart transplantation and, in particular, after coronary balloon angioplasty and endovascular stenting. Generally, one-third of patients undergoing any one of these procedures will develop restenosis within 6 months. Accordingly, a therapeutic dosage form designed to release a water-insoluble paclitaxel derivative for a time period of at least 2 months, preferably for about 4 months, and more preferably for about 6 months, is contemplated.

Some exemplary therapeutic dosage forms which provide such sustained release will now be described.

### A. Implantable Medical Devices

The water-insoluble paclitaxel derivatives are contemplated for use as a coating for implanted medical devices, such as tubings, shunts, catheters, artificial implants, pins, electrical implants such as pacemakers, and especially for arterial or venous stents, which will be described in detail below. In these devices, the derivative can be bound to an implantable medial device or can be passively adsorbed to the surface of the device. For example, metal devices can be coated with polymer-drug solution by dipping the device in the solution or by spraying the device with the solution.

### 1. Stents

In a preferred embodiment, the medical device is a radially expandable stent which carries the water-insoluble paclitaxel derivative to a target site in a vessel. Endovascular stents are well known in the art and are commercially available. Such stents are typically made of a biocompatible metal, such as such as nickel-titanium alloys and stainless steel, or are composed of polymer, as has been described in, for example, in U.S. Patent Nos. 5,163,952 and 5,603,722.

For purposes of the present invention, a metal stent would be used as a support stent for a polymer sleeve, sheath or member which contains the water-insoluble paclitaxel derivative. A variety of polymers suitable for incorporation of a water-insoluble paclitaxel derivative and for formation of a coating layer or sleeve onto the metallic stent are known to those of skill in the art, and in particular methacrylate and acrylate polymers are suitable.

The metal support stent can take a variety of forms, and is generally suitable for implantation into a body lumen in a collapsed or small-diameter condition and for expansion to a larger diameter condition upon placement at the site to be treated. Stents known in the art and suitable for use in the present invention include pressure-expandable stents, self-expanding stents and stents which expand in response to an applied stimulus, such as heat. An exemplary pressure-expanding stent is described in United States Patent Nos. 4,776,337 and 4,733,665 to Palmaz. Pressure-expandable stents are typically radially expanded by means of a balloon angioplasty catheter, as is known in the art. Self-expanding stents, such as the stent described by Gianturco in United States Patent No. 4,580,568 and by Wallsten in United States Patent No. 4,544,771, radially expand due to the inherent spring tension of the stent. The stents expand to a larger diameter after being released from a constraining force which restricts it to a smaller diameter. Another sort of self-expanding stent includes stents made of shape-memory material, such as nitinol or shape-memory polymers described by Froix in United States Patent No. 5,163,952.

Stents prepared of polymer and loaded with the water-insoluble paclitaxel derivative are also contemplated. In particular, stents composed of acrylate and methacrylate-based polymers, such as the shape-memory polymers described by Froix in United States Patent No. 5,163,952, loaded with the drug are suitable.

In support of the present invention, studies were conducted using a metal support stent which carried one or more polymer sleeves about its outer circumference. The polymer sleeve(s) contained the water-insoluble paclitaxel derivative, 7-hexanoyl taxol. Preparation of a polymer sleeve containing 7-hexanoyl taxol is described in Example 2. The exemplary polymer sleeves were prepared from an acrylate/methyacrylate monomer mixture and polymerized using a methacrylate crosslinker. 7-hexanoyl taxol was added to the polymer by contacting the polymer with a concentrated solution of the 7-hexanoyl taxol in a suitable organic solvent.

As will be described below, the drug-loaded, support scent carrying one or more polymer sleeves was deployed in the coronary arteries of pigs for evaluation and was compared to the same stent carrying paclitaxel.

The polymer composition described in Example 2 is also suitable for use in preparing a polymer stent capable of carrying the water-insoluble paclitaxel derivative. The polymer formulation is also suitable to apply a thin polymer coating to either a metal or polymer stent for purposes of carrying the water-insoluble paclitaxel derivative.

### IV. In vivo Testing

In studies performed in support of the invention a stent carrying the water-insoluble derivative, 7-hexanoyl taxol, was prepared and inserted into the arteries of pigs. The stent was composed of a metal support stent carrying a polymer stent thereon and is depicted in Fig. 3A-3C. Fig. 3A shows the metal support stent 10 alone in an expanded, large diameter condition. The stent is composed of unit cells, such as unit cells 12, 14, 16, joined in a radial direction to form a plurality of unit cells 18. Each unit cell is expandable to move the stent from a small-diameter condition, for insertion into a body lumen, to a large-diameter condition, for deployment into the body lumen. Support stent 10 as shown is composed of four pluralities of unit cells, 18, 20, 22 and 24. The pluralities of unit cells are joined radially by a connecting segment, such as connecting segments 26a, 26b, 26c, which join pluralities 18, 20; 20, 22; and 22, 24, respectively. As can be appreciated, the stent can be composed of any number of pluralities to give any desired stent length, and the dimensions of each unit cell can readily be varied to determine stent length and diameter. The stent in regions which correspond to each plurality of unit cells, is relatively rigid compared to the regions between each plurality and corresponding to the connecting segments. This is an important feature of the stent, since the more flexible regions corresponding to the connecting segments gives better flexibility and tractability to the stent for easier navigation and placement in vessels. The stent of Fig. 3A is described in detail in co-owned PCT Publication No. WO 99/49811.

Fig. 3B shows the metal stent of Fig. 3A with a continuous polymer sheath 30 encasing the metal support stent. The outer polymer sleeve is prepared, for example, as set forth in Example 2, and contains the 7-hexanoyl taxol. or other compound. The sleeve is carried coaxially about the outer circumference of the support stent and takes the form of a flat sheet rolled into a cylindrical or tubular shape by overlapping the edges 32, 34 of the sheet. It will be appreciated that the initial configuration of the tubular member is not limited to a flat sheet, but can also be prepared from an extruded tube-form.

Fig. 3C illustrates another embodiment of a stent for use in the invention, where stent 40 is composed of metal stent 10 of Fig. 3A and includes a plurality of polymeric materials about the outer circumference. Stent 10 has four rigid regions which correspond to the unit cell pluralities 18, 20, 22, 24 (see Fig. 3A). By "rigid" it is meant that in this region of the stent, flexure in the radial direction is minimal, especially when compared to the radial flexure of the regions corresponding to where the connecting segments join the rigid regions. These flexible regions are identified in Fig. 3C as regions 42a, 42b, 42C. The polymeric materials are disposed coaxially about the outer stent surface only in the rigid stent regions, as are polymeric materials 44, 46, 48, 50, leaving the flexible regions 42a, 42b, 42c, exposed or uncovered. This positioning of the polymeric materials offers the advantage of carrying a polymeric material for administration of a therapeutic compound, while maintaining the flexibility offered by the articulating stent. The configuration also overcomes problems associated with drape and sag of the polymer member when it covers the regions of flexure (as in the embodiment of Fig. 3B), as structural support for the polymer is less adequate than in the rigid regions of the support stent.

Figs. 4A-4C illustrate another exemplary support stent suitable for use in the invention. A metal support stent 60 is shown in Fig. 4A in its small-diameter, unexpanded condition. Stent 60 has two regions of rigidity, 62, 64, where flexure in the radial direction is minimally possible. The two rigid regions are joined by one or more connecting segments, such as segments 66a, 66b, and define a flexible stent region 68. The same stent is shown in Fig. 4B in its larger diameter, expanded condition, where the rigid regions 62.64 and the flexible region 68 are clearly indicated. Stent 60 includes at least one polymeric material disposed about one or more of the rigid stent regions. As shown in Fig. 4C, polymeric materials 70, 72 cover rigid regions 62, 64, respectively, leaving flexible region 68 uncovered and exposed.

Another example of a support stent with polymeric materials is illustrated in Figs. 5A-5C. Here the support stent 80 in its small diameter condition is shown in Fig. 5A where rigid stent regions 82, 84 are joined by one or more connecting segments 86a, 86b, which define a region of flexibility 88. The stent in its large diameter, expanded condition after placement in a vessel is shown in Fig. 5B. The stent with polymeric materials covering the rigid stent regions is shown in Fig. 5C, where polymeric materials 90, 92 are positioned over rigid regions 82, 84, respectively.

The support stent is composed of a biocompatible material, and suitable materials include metals, such as stainless steel, tungsten, titanium, gold, platinum and tantalum, alloys of these materials and others, as well as shape-memory alloys, high strength thermoplastic polymers, copolymers, including shape-memory polymers. Shape-memory copolymers including homopolymers and copolymers are contemplated.

The polymeric materials are composed of any biocompatible polymer, such as polyamides, polyimides, silicones and fluorinated polyolefins. A preferred fluorinated polyolefin is polytetrafluoroethylene, which can be either biaxially oriented polytetrafluoroethylene or uniaxially oriented polytetrafluoroethylene. In one embodiment of the invention, the polymeric material is prepared from between 10-98 weight percent of acrylate monomer, more preferably greater than 40 weight percent, and between 2-40 weight percent of a polyalkyleneoxide monomer, more preferably between about 10-30 weight percent. An exemplary polyalkyleneoxide monomer is polyethylene oxide monomethyl ether monomethacrylate. In another embodiment, added to the acrylate monomer and the polyalkyleneoxide monomer is between 3-30 weight percent of a methacrylate monomer. Polymers formed of these monomers are described in more detail in a copending, co-owned application, which is incorporated by reference herein.

The polymeric material is formed into a tubular configuration, either by fabrication or extrusion directly into a cylindrical form or by wrapping a polymer sheet into a cylindrical configuration. The polymeric materials are secured in an unexpanded diameter to the support stent by a mechanical means, such as by ultrasonic welding, resistive heating and laser irradiation. Alternatively, the polymer tubular material is secured to the support stent in an unexpanded diameter by a biocompatible adhesive, such as a fluorinated thermoplastic polymer adhesive. Examples of fluorinated thermoplastic include fluorinated ethylene/propylene copolymers, perfluoroalkoxy fluorocarbons, ethylene/tetrafluoroethylene copolymers, fluoroacrylates, and fluorinated polyvinyl ethers. It is also possible that the polymeric material has sufficient inherent elasticity to remain secured to the support stent in its small, unexpanded diameter and for expansion with the support stent.

The therapeutic agent can be incorporated into the polymeic material by a variety of methods. For example, the polymeric materials can be soaked in a solution containing the agent to imbibe the drug into the polymer. The solvent can then be removed by heating or reducing pressure. The polymeric materials can be formed by dissolving the polymer in a solution containing the agent and allowing the solvent to evaporate to form a polymer sheet, which is then cut into sizes suitable for formation of the polymeric materials. Other methods are apparent to those of skill in the art.

In another embodiment of the invention, the polymeric material carries two therapeutic agents, where in a preferred embodiment, the first agent is paclitaxel or a derivative of paclitaxel and the second agent is any of those recited above, preferably camptothecin. colchicine, dexamethasone, melphalan, econozole or tamoxifen.

In studies performed in support of the invention, polymeric materials containing 7-hexanoyl taxol were prepared from an acrylate/methyacrylate monomer mixture and polymerized using a methacrylate crosslinker, as described in Example 2. 7-hexanoyl taxol was incorporated into the polymer by contacting the polymer with a concentrated solution of the 7-hexanoyl taxol in a suitable organic solvent. Such polymeric materials, and polymeric materials similarly formed but with paclitaxel rather than 7-hexanoyl taxol, were used in combination with a support stent, as illustrated in Fig. 3C. The test and control stents were placed in the coronary arteries of pigs, as described in Example 3, and were left *in vivo* for 28 days. A metal stent not carrying a polymeric material was also placed into an-artery. At the time of insertion of the test and control stents, the coronary artery was characterized using a computer-based coronary angiography analysis system (Umans, V.A., *et al., JACC* 21(6):1382-1390, (1993)). Boundaries of a selected coronary artery segment were detected automatically from optically magnified and video-digitized regions of interest. The catheter used for insertion of the stents was used as a scaling device to determine the dimensions of the artery at the site of implantation. The original vessel diameter at the time of implantation was determined.

After the 28 day test period, the arteries were then explanted from the pig and pressure fixed for morphometric analysis. The minimal lumen diameter of the vessel after the treatment period was found by determining the smallest lumen diameter in the region of stent placement. The percent stenosis was taken as one minus the stented vessel's minimum lumen diameter divided by the diameter of an unstented reference vessel time one-hundred. The percent intimal growth was also determined from the following equation: 1-[(stented vessel's minimum lumenal diameter)/(diameter of stented portion prior to stent placement)]*100. The balloon to artery ratio was also determined as a measure of the degree of distension of the vessel by the balloon. The results are shown in Table 2.

**Table 2**

| **Stent Configuration** | **Stent Location** | **Balloon to Artery Ratio** | **% Diameter Stenosis** | **% Intimal Growth** |
|---|---|---|---|---|
| metal support/polymer segments with 7-hexanoyl taxol | right coronary artery | 1.04 | 4 | -7.1 |
| metal support /polymer segments with 7-hexanoyl taxol | left circumflex artery | 0.98 | 16 | 2.3 |
| metal support /polymer segments with paclitaxel | right coronary artery | 1.16 | 34 | 29.5 |
| metal support stent (no drug) | left anterior descending artery | 1.16 | 22 | 73.2 |
| metal support stent (no drug) | right coronary artery | 1.11 | 27 | 73.2 |

As can be seen from the data in Table 2, arteries treated with stents containing 7-hexanoyl taxol had the lowest percent stenosis. Compared to the stent containing paclitaxel, the stent with 7-hexanoyl taxol resulted in about a two-fold reduction in percent stenosis, for one case, and in the other case, about an 8-fold reduction in percent stenosis. The percentage intimal growth was also significantly better for the stents carrying 7-hexanoyl taxol, when compared to the stent carrying paclitaxel and to the control metal stents.

The finding that *in vivo* 7-hexanoyl paclitaxel is significantly more effective in treating restenosis than paclitaxel was surprising in view of the *in vitro* cytotoxicity results discussed above in Table 1. In those tests, in some cell lines the 7-hexanoyl taxol was more effective, however in other cell lines the paclitaxel was more effective. Based on this, the considerable improvement achieved with the 7-hexanoyl taxol *in vivo* is unexpected.

In another study in support of the invention, stents were prepared as described in Examples 2 and 3. The polymeric materials carried either 7-hexanoyl taxol, paclitaxel or 7-xylosyltaxol. 7-xylosyltaxol is a sugar derivative of paclitaxel and has been described in WO 96/11683. The 7-xylosyl taxol, having the hydrophilic sugar moiety, is more water-soluble than paclitaxel. The stents were placed in coronary arteries of pigs, according to the procedure described above. After the test period, the percent diameter stenosis and neointimal thickness of the arteries were determined. The percentage of diameter stenosis was determined by two procedures, via quantative coronary angiography (QCA) and via morphometric evaluation of the vessels. The later procedure is described in Example 3. The former procedure, QCA, was performed according to the procedure of Umans, *et al*. described above, with lumen dimensions determined prior to explanting the vessels for morphometric analysis. The results are reported in Table 3.

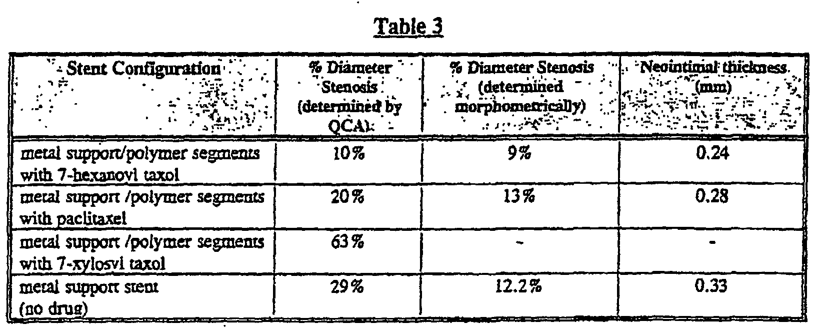

The data in Table 3 shows that the water-insoluble paclitaxel derivative resulted in considerably lower percent diameter stenosis than the water-soluble derivative, 7-xylosyltaxol. The study also supports the data in Table 2, indicating that the water-insoluble derivative achieved lower percent stenosis and less intimal thickening than pactitaxel.

In yet another study performed in support of the invention, metal support stents carrying a plurality of polymer sleeves as depicted in Fig. 3C were prepared and tested in humans at risk for restenosis. Polymer sleeves prepared as described in Example 2 were loaded with 800 µg of 7-hexanoyl taxol. Metal stents, as depicted in Fig. 3A and as described in detail in co-owned PCT Publication No. WO 99/49811, were used as the support structure for the drug-loaded polymer sleeves. The stent sizes were between 13-17 mm in length and from 3.0-3.5 mm in diameter (after expansion) depending on the lesion and the vessel size. Stents of 13 mm in length carried 4 polymer sleeves, each loaded with 800 µg of 7-hexanoyl taxol. The stents of 17 mm in length carried 5 polymer sleeves, each loaded with 800 µg of 7-hexanoyl taxol.

Thirty-one patients at risk for restenosis and having vessel lesions were randomized into two treatment groups of 16 individuals to receive the stent carrying the drug-loaded polymer and 15 individuals to receive a metal support stent with no polymer sleeve or drug. Of the 31 patients, 46% had type A lesions, 54% had type B2 and C lesions, using the American College of Cardiology/American Heart Association (ACC/AHA) classification of lesion morphology (Kastrati, A., *et al. Circulation* 100(12):1285, (1999)).

The test and control stents were inserted into the patients via balloon catheter, and all stents were successfully placed in the selected vessel of each patient, as determined by intravascular ultrasound (IVUS; Erbel, R. *et al. Coron. Artery Dis.* 10(7):489, (1999)). For 3-8 months following stent insertion, each patient was monitored clinically, angiographically and by intravascular ultrasound (IVUS). Quantative coronary angiography (described above) and quantative coronary ultrasound were performed by an independent lab in 25 of the patients to verify the findings.

Table 4 summarizes the data from the human trial. The average minimum lumen diameter (MLD) in mm before insertion of the test stent and the minimum lumen diameter in mm after insertion and expansion of the stent for the two test groups are shown, as is the average minimum lumen diameter in mm at the follow-up visit (between 3-8 months). The percent diameter stenosis taken as the [MLD(post insertion) - MLD(follow-up)]/MLD(post insertion) was determined.

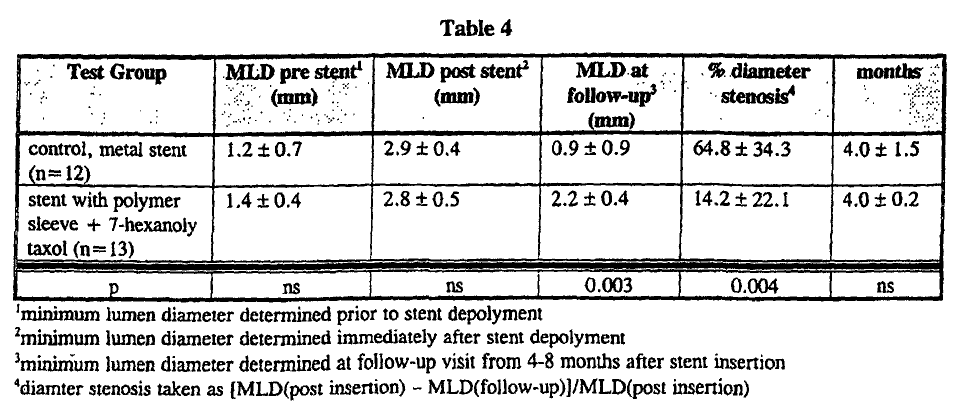

As seen in Table 4, the patient test populations prior to stent insertion had similar averaged minimum lumen diameters of 1.2 ± 0.7 mm and 1.4 ± 0.4 mm in vessels normally of between 3-3.5 mm diameter. After stent deployment, the vessels in the patients of each test group were similarly expanded, to 2.9 ± 0.4 mm for the control test group and 2.8 ± 0.5 mm for the group receiving the stent in accord with the invention. At the follow-up visit for each patient the minimum lumen diameter was again determined (by IVUS and/or angiographically) and the averaged values for each test group are shown in Table 4. The group receiving a metal stent with no water insoluble paclitaxel derivative had an average minimum lumen diameter of 0.9 ± 0.9 mm, whereas the patients treated with the drug-loaded stent of the invention had a minimum lumen diameter of 2.2 ± 0.4 mm. The patients treated with the drug-loaded stent showed nearly total absence of intimal proliferation at the lesion site, including the end regions of the lesion. In contrast, the patients in the control group had significant restenosis.

In this study, the stents contained either 3.2 mg of 7-hexanoyl taxol or 4.0 mg 7-hexanoyl taxol, depending on the length of the stent. This amount of drug is administered locally to the treatment site over a period of at least 2 months, more preferably over 3 months, and most preferably over a 6 month period. The unique combination of a high drug loading achievable with selected polymers and the insoluble nature of the paclitaxel derivative provide a means to ensure sufficient drug over the time frame needed (at least 2-6 months or 3-6 months).

Table 5 compares the dose of 7-hexanoyl-taxol needed for treatment and/or prevention of restenosis when administered from a stent loaded with 4 mg of 7-hexanoyl taxol with the dosage of paclitaxel and docetaxel used in chemotherapy. For purposes of comparison it was assumed that the 4 mg stent dose is released as a bolus.

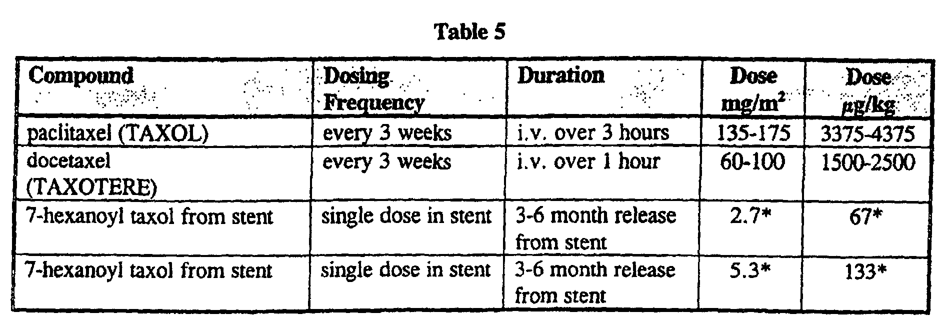

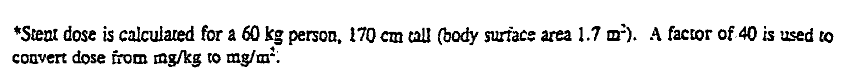

Table 5 shows that the dose of the water-insoluble taxol derivative is significantly less than that used in chemotherapy. Accordingly, in one embodiment of the invention, the dose of the water-insoluble taxol derivative used for treatment/prevention of restenosis is 10-fold, preferably 25-fold, more preferably 50-fold less, still more preferably 100-fold lower than that used for chemotherapy. The daily dose of 7-hexanoyl taxol estimated for treatment of restenosis is 0.02-0.03 mg/m² using a stent designed to deliver 2.7 mg/m² for 3-6 months.

From the foregoing, it will be appreciated now various features of the invention are met. The composition of the invention, composed of a water-insoluble paclitaxel derivative, is incorporated into a suitable polymeric material for administration to a target lumen. In one preferred embodiment, the derivative is incorporated into a stem which is placed in a lumen for prevention of restenosis following angioplasty. Suitable dosages of water-insoluble derivatives can be discerned by those of skill in the art using guidance from the effective dosages of paclitaxel and similar compounds.

### V. Examples

The following examples further illustrate the features of the invention and in no way are to be considered as limiting to the scope and spirit of the invention.

### Example 1

### In vitro Cytotoxicity

A suspension of each cell line which was diluted according to the particular cell type and the expected target cell density (5,000-40,000 cells per well based on cell growth characteristics) was added by pipet (100 µL) into a 96-well microtiter plate. The cells were allowed a pre-incubation period of 24 hours at 37°C for stabilization. At the end of the pre-incubation period (T₀), dilutions of 7-hexanoyl taxol or paclitaxel were added in 100 µL aliquots to the microtiter plate wells. The cells were incubated in the presence of the drug for 48 hours under a 5% CO2 atmosphere at 100% humidity. The cells were assayed using the sulforhodamine B assay. A plate reader was used to read the optical densities.

The LC₅₀ values were taken as the concentration of compound where 100 x (T-T₀)/T₀ = -50. The results are shown in Table 1.

### Example 2

### Polymer Sleeve Preparation

The following mixture of monomers was weighed into a suitable container: 60.1% butyl acrylate (Aldrich Chemical, St. Paul MN); 30% polyethylene oxide monomethyl ether monomethacrylate (MW 1000 daltons)(NOF Corp., Tokyo Japan); and 9.8% methylmethacrylate (Aldrich Chemical). 0.05% of hexane diol dimethacrylate (Aldrich Chemical), a cross-linker, and 0.10% of Darocur® 1173 (E. Merck, Dramstadt, Germany), a photoinitiator, were added to initiate polymerization. The monomers are mixed together, purged with nitrogen and then polymerized between glass plates to form thin films having a thickness of approximately 0.14 mm. The copolymer film is cut into the desired size for formation of the sleeve.

A solution of 7-hexanoyl taxol in dimethylformamide was prepared, with the 7-hexanoyl taxol just below the solubility limit in the solvent at room temperature. A known quantity of the solution was placed on the polymer sleeve using a micropipet and allowed to absorb into the copolymer.

### Example 3

### In vivo Testing of Polymer Stent Containing 7-Hexanoyl Taxol

A polymer sleeve, prepared as described in Example 2, was placed about a metal, corrugated stent. The polymer sleeve contained 1500 µg of 7-hexanoyl taxol. As a comparative control, a similar stent was prepared to contain 1500 µg of paclitaxel.

The two-drug loaded stents and two control metal stents with no polymer sleeve were placed into the coronary arteries of healthy Domestic Farm Swine pigs (Pork Power, Inc.) by conventional techniques using a commercially available catheter (Advanced Cardiovascular Systems). The stents were imaged during and after the insertion procedure to ensure proper placement using conventional angiographic imaging techniques. The metal control stents were placed in two different pigs, in one pig the stent was positioned in the left anterior descending artery and in the other pig in the right coronary artery. The comparative control stent containing paclitaxel was placed in one animal in the right coronary artery. The test stents containing 7-hexanoyl taxol were placed in two pigs, one stent in the right coronary artery and the other stent in the other pig in the left circumflex artery.

Twenty-eight days after placement the pigs were euthanized and the heart and coronary arteries explanted. The arteries were pressure fixed for morphometric analysis to determine the percent diameter stenosis and percent intimal growth. The percent diameter stenosis was taken as 1-[(stented vessel's minimum lumenal diameter)/(diameter of unstented reference vessel)]*100. The percent intimal growth was taken as 1-[(stented vessel's minimum lumenal diameter)/(diameter of stented portion prior to stent placement)]*100. The balloon to artery ratio was also determined as a measure of the degree of distension of the vessel by the balloon. The results are shown in Table 2.

Although the invention has been described with respect to particular embodiments, it will be apparent to those skilled in the art that various changes and modifications can be made without departing from the invention.

## Claims

1. A composition for administration of a paclitaxel derivative, comprising a paclitaxel derivative having a water solubility less than that of paclitaxel, and a polymeric material containing the paclitaxel derivative, wherein the polymeric material is in a form selected from the group consisting of a coating; a sleeve; a sheath or an implantable medical device.

2. The composition according to claim 1, wherein the paclitaxel derivative is a compound derivatized at the 2', 10 or 7 position of taxol.

3. The composition according to claim 2, wherein the paclitaxel derivative is 7-hexanoyl taxol.

4. The composition according to any one of claims 1-3, wherein the medical device is a stent.

5. The composition according to any one of claims 1-4, wherein the polymeric material includes an acrylate or methacrylate polymer or a polyalkyleneoxide.

6. The composition according to any one of claims 1-5, wherein the polymeric material contains the paclitaxel derivative in particulate form.

7. The composition according to any one of claims 1-5, wherein the polymeric material is comprised of greater than about 40 weight percent of an acrylate monomer and between about 3-30 weight percent of a polyalkyleneoxide monomer, said monomers, when polymerized, forming a copolymer having the paclitaxel derivative incorporated therein.

8. The composition according to claim 7, wherein the polymeric material further includes between 3-30 weight percent of a methacrylate monomer which is copolymerized with the acrylate monomer and the polyalkyleneoxide monomer.

9. The composition according to claim 7 or claim 8, wherein the acrylate monomer is butyl acrylate.

10. The composition according to any one of claims 7-9, wherein the polyalkyleneoxide monomer is polyethylene oxide monomethyl ether monomethacrylate.

11. A composition according to any one of claims 1-10 for use in preventing or treating restenosis.

12. Use of a composition according to any one of claims 1-10 for the manufacture of a medicament for the treatment or prevention of restenosis.

13. The composition of claim 1 wherein the polymeric material is a coating disposed upon a surface of an implantable medical device.

14. The composition of claim 13 wherein the medical device is a stent.

## Patentansprüche

1. Zusammensetzung zur Verabreichung eines Paclitaxelderivats, umfassend ein Paclitaxelderivat mit einer Wasserlöslichkeit unterhalb der von Paclitaxel und ein Polymermaterial, das das Paclitaxelderivat enthält, wobei das Polymermaterial in einer Form vorliegt, ausgewählt aus der Gruppe bestehend aus einer Beschichtung, einer Hülse, einer Hülle oder einer implantierbaren medizinischen Vorrichtung.

2. Zusammensetzung nach Anspruch 1, wobei das Paclitaxelderivat eine Verbindung ist, die an der 2'-, 10- oder 7-Position von Taxol derivatisiert ist.

3. Zusammmensetzung nach Anspruch 2, wobei das Paclitaxelderivat 7-Hexanoyltaxol ist.

4. Zusammensetzung nach einem der Ansprüche 1 bis 3, wobei die medizinische Vorrichtung ein Stent ist.

5. Zusammensetzung nach einem der Ansprüche 1 bis 4, wobei das Polymermaterial ein Acrylat- oder Methacrylatpolymer oder ein Polyalkylenoxid einschließt.

6. Zusammensetzung nach einem der Ansprüche 1 bis 5, wobei das Polymermaterial das Paclitaxelderivat in Partikelform enthält.

7. Zusammensetzung nach einem der Ansprüche 1 bis 5, wobei das Polymermaterial aus mehr als etwa 40 Gewichtsprozent eines Acrylatmonomers und zwischen etwa 3 und 30 Gewichtsprozent eines Polyalkylenoxidmonomers zusammengesetzt ist, wobei die Monomere, wenn sie polymerisiert sind, ein Copolymer bilden, in dem das Paclitaxelderivat inkorporiert ist.

8. Zusammensetzung nach Anspruch 7, wobei das Polymermaterial weiterhin zwischen 3 und 30 Gewichtsprozent eines Methacrylatmonomers umfasst, das mit dem Acrylatmonomer und dem Polyalkylenoxidmonomer copolymerisiert ist.

9. Zusammensetzung nach Anspruch 7 oder 8, wobei das Acrylatmonomer Butylacrylat ist.

10. Zusammensetzung nach einem der Ansprüche 7 bis 9, wobei das Polyalkylenoxidmonomer Polyethylenoxidmonomethylethermonomethacrylat ist.

11. Zusammensetzung nach einem der Ansprüche 1 bis 10 zur Verwendung bei der Vorbeugung gegen oder der Behandlung von Restenose.

12. Verwendung einer Zusammensetzung nach einem der Ansprüche 1 bis 10 zur Herstellung eines Arzneimittels zur Behandlung von oder zur Vorbeugung gegen Restenose.

13. Zusammensetzung nach Anspruch 1, wobei das Polymermaterial eine Beschichtung ist, die auf einer Oberfläche einer implantierbaren medizinischen Vorrichtung aufgetragen ist.

14. Zusammensetzung nach Anspruch 13, wobei die medizinische Vorrichtung ein Stent ist.

## Revendications

1. Composition pour l'administration d'un dérivé de paclitaxel, comprenant un dérivé de paclitaxel ayant une hydrosolubilité inférieure à celle du paclitaxel et un matériau polymérique contenant le dérivé de paclitaxel, dans laquelle le matériau polymérique est sous une forme choisie parmi le groupe comprenant un enrobage, un manchon, une gaine ou un dispositif médical implantable.

2. Composition selon la revendication 1 dans laquelle le dérivé de paclitaxel est un composé dérivatisé en position 2', 10 ou 7 du taxol.

3. Composition selon la revendication 2 dans laquelle le dérivé de paclitaxel est le 7-hexanoyle taxol.

4. Composition selon l'une quelconque des revendications 1 à 3, dans laquelle le dispositif médical est un stent.

5. Composition selon l'une quelconque des revendications 1 à 4, dans laquelle le matériau polymérique inclut un polymère d'acrylate ou de méthacrylate ou un oxyde de polyalkylène.

6. Composition selon l'une quelconque des revendications 1 à 5, dans laquelle le matériau polymérique contient le dérivé de paclitaxel sous forme particulaire.

7. Composition selon l'une quelconque des revendications 1 à 5, dans laquelle le matériau polymérique comprend plus qu'environ 40 % en poids d'un monomère d'acrylate et entre environ 3 et 30 % en poids d'un monomère d'oxyde de polyalkylène, lesdits monomères, lorsqu'ils sont polymérisés, formant un copolymère intégrant le dérivé de paclitaxel.

8. Composition selon la revendication 7, dans laquelle le matériau polymérique inclut en outre entre 3 et 30 % en poids d'un monomère de méthacrylate qui est copolymérisé avec le monomère d'acrylate et le monomère d'oxyde de polyalkylène.

9. Composition selon la revendication 7 ou la revendication 8, dans laquelle le monomètre d'acrylate est de l'acrylate de butyle.

10. Composition selon l'une quelconque des revendications 7 à 9, dans laquelle le monomère d'oxyde de polyalkylène est du polyéthylène oxyde monométhyle éther monométhacrylate.

11. Composition selon l'une quelconque des revendications 1 à 10 pour une utilisation pour la prévention ou le traitement de la resténose.

12. Utilisation d'une composition selon l'une quelconque des revendications 1 à 10 pour la fabrication d'un médicament pour le traitement ou la prévention de la resténose.

13. Composition de la revendication 1, dans laquelle le matériau polymérique est un enrobage placé sur une surface d'un dispositif médical implantable.

14. Composition de la revendication 13, dans laquelle le dispositif médical est un stent.
